Europäisches Patentamt

European Patent Office

Office européen des brevets

(11) Publication number: **0 272 656**
**A2**

# (12) EUROPEAN PATENT APPLICATION

(21) Application number: **87118870.2**

(22) Date of filing: **18.12.87**

(51) Int. Cl.⁴: **A61M 1/28**

(30) Priority: **19.12.86 IT 3283686 U**

(43) Date of publication of application:
**29.06.88 Bulletin 88/26**

(84) Designated Contracting States:
**AT BE CH DE ES FR GB GR IT LI LU NL SE**

(71) Applicant: **Buoncristiani, Umberto**
**Via G. Brufani 23**
**I-06100 Perugia(IT)**

Applicant: **Buoncristiani, Renata**
**Via G. Brufani 23**
**I-06100 Perugia(IT)**

(72) Inventor: **Buoncristiani, Umberto**
**Via G. Brufani 23**
**I-06100 Perugia(IT)**
Inventor: **Buoncristiani, Renata**
**Via G. Brufani 23**
**I-06100 Perugia(IT)**

(74) Representative: **Grams, Klaus Dieter, Dipl.-Ing.**
**et al**
**Patentanwaltsbüro Tiedtke-Bühling-Kinne-**
**Grupe-Pellmann-Grams-Struif-Winter-Roth**
**Bavariaring 4**
**D-8000 München 2(DE)**

(54) Disinfection auxiliary device for peritoneal dialysis.

(57) A disinfecting auxiliary device for continuous ambulatorial peritoneal dialysis comprises a three way fitting, which can be fixed by a first branch to the end of the outlet pipe of a supply bag for fresh dialysis solution, a second branch being connectable to the end portion of the standard Y shaped fitting mounted to the catheter implanted in the patient abdomen and the third or side branch is connectable to a supply bag for liquid disinfectant.

Fig. 1

## Disinfection auxiliary device for peritoneal dialysis

The present invention relates to the field of the peritoneal dialysis and more particularly to an auxiliary device useful for the phase of connecting and filling of the peritoneal cavity with a bag containing fresh dialysis solution.

The peritoneal dialysis is a technique for the depuration of the blood of an intoxicated patient (mainly owing to uremic toxins accumulated as a consequence of a compromised renal functionality) characterized by the transfer of the toxins (either uremic or of any other nature) through the peritoneal membrane (covering the internal walls of the abdomen and the bowels therein contained) to a solution filling the abdominal cavity and wetting the peritoneal membrane. Such a solution tends to reach an equilibrium condition with the blood as regards the concentration of the several toxins and thus must be often renewed. More particularly, in the embodiment called CAPD (continous ambulatorial peritoneal dialysis) the dialysis solution is renewed four times per day and continuatively every day. Consequently a high number of weeikly substitution operations and a high number as well of connecting and disconnecting operations between the catheter permanently implanted in the patient abdomen and the outlet pipe of the bag containing the fresh dialysis solution result, it in turn leading to a high incidence of bacterial contamination at the level of the connecting and disconnecting points with the attendant bacterial peritonitis.

In order to try to reduce such a drawback an Y shaped connecting fitting has been adopted, by which a disinfectant can be used so as to come into conect with the end portion of the fitting which is connected and disconnected with respect to the said catheter, before the flow therethrough of the fresh dialysis solution, it being then discharged to the outside through the side branch of the said fitting.

However the problem still exists as regards the distal 02 remote end of the fitting. To date, in order to solve this problem by casuing the disinfectan is causedt to return through the outlet pipe of the Y shaped fitting and fill it starting from a basin containing the disinfectant, within which the said outlet pipe is preventively dipped: the return of the disinfactant takes place by diffusion and mainly owing to a suking effect induced by a sort of pumping action provided by a squeezing and releasing the flexible end portion of the said outlet pipe.

Both those filling mechanisms are however alcatory and anything but safe, especially the second one mostly depends on the ability of the patient. Consequently the peritonitis complication, although of markedly reduced frequency, is still present and does heavily condition the success and diffusion of CAPD.

The main purpose of the present invention is that of eliminating the above problem.

To this end an auxiliary device is provided, adapted to be mounted to the outlet of a supply bag containing fresh dialysis solution and connectable to the inlet of the standard Y shaped fitting fixed to the cathether implanted in the patient abdomen, said auxiliary device being characterized by comprising a three way fitting one branch of which can be fixed to the outlet pipe of the said supply bag, a check valve being inserted in said one branch, an opposite second branch connectable to the inlet of said Y shaped fitting and a third, side branch connectable to a supply of disinfectant, said second and third branches being provided with closing plugs.

As it will be appreciated from the following detailed description, the auxiliary device of the present invention permits not only the said Y shaped fitting connected to the catheter to be fully washed and filled with disinfectant, but also the end part of the auxiliary device connected to said Y shaped fitting to be washed with disinfectant, thus avoiding any risk of bacterial contaimination before the fresh dialysis solution is fed to the patient abdomen.

Another advantageous feature of the present invention resides in that the disinfectant is fully disposed and no traces thereof remain in the circuit leading from the bag contaning the fresh dialysis solution to the peritoneal cavity, the latter being thus filled only with pure and fresh dialysisy solution.

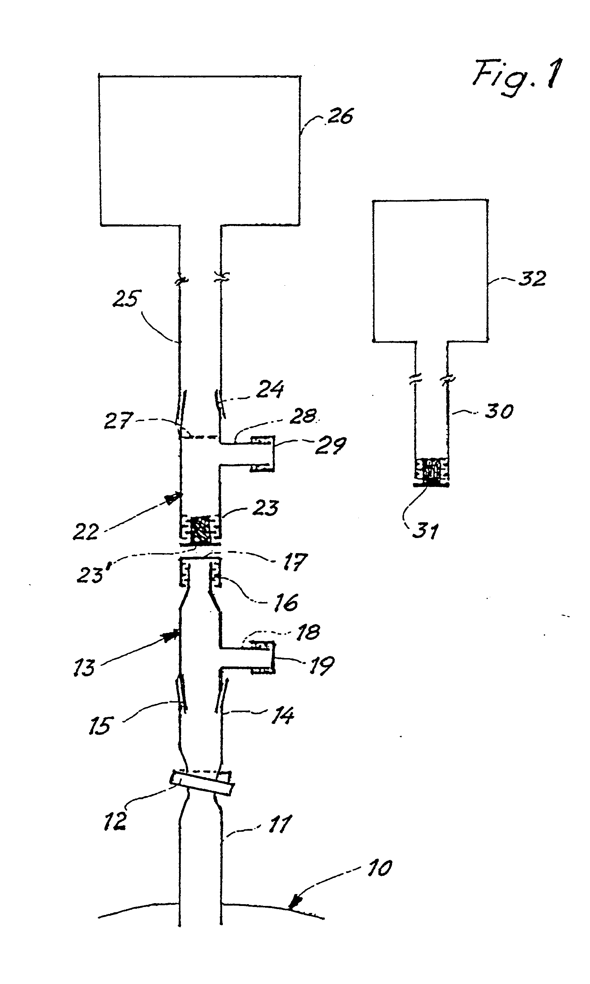

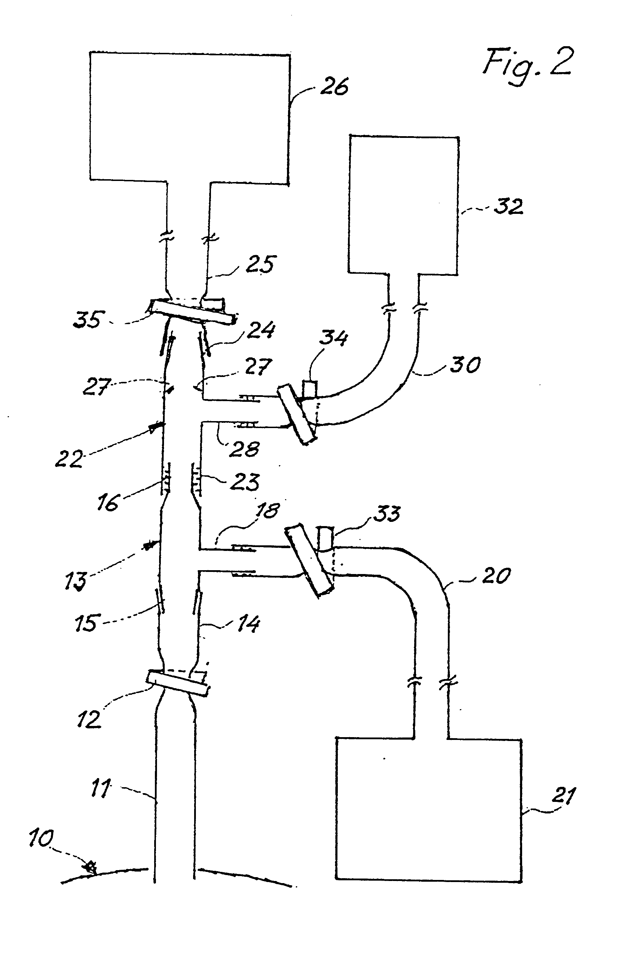

In the figures 1 and 2 of the enclosed drawings schematic views of one embodiment of the auxiliary device of the invention are shown, corresponding to two operative conditions respectively.

In the figures reference 10 indicates the outer surface of the patient abdomen, in which a catheter 11 is permanently implanted.

Reference 12 indicates a clamping device, normally manually actuated, of the type well known for instance of the clamping and adjustable opening of flexible pipes for hospital or medical use (e.g. in the phleboclysis). An Y shaped fitting, indicated as a whole by the reference 13, is also permanently mounted to the other or distal end portion 14 of the catheter and comprises a first branch 15, connected in a per se known manner (for instance by forced coupling) to the said end portion 14; the second or opposite branch 16 of the Y fitting is closed by a plug or cap 17, which can be removed and ensures the tight seal closure of this end portion. A third branch 18 of the Y shaped fitting 13 is a sidewise protruding and is closed by a similar plug or cap 19.

During the use, this branch 18 is adapted to be connected through a pipe 20 (fig. 2) to a disposal bag 21.

The auxiliary device of the present invention, generically shown in the figures by the reference 22, comprises a three way fitting adapted to be mounted to the outlet pipe 25 of a supply 26 of fresh dialysis solution which is preferably in form of a flexible bag containing a predetermined amount of dialysis solution.

The way or branch end 24 is connected to the said outlet pipe 25, a chek valve 27, preferably in form of a fracture membrane, being positioned within the branch 24 to prevent any liquid flow therethrough unless the membrane 27 is broken.

The opposite way or branch end 23 in the inoperative condition is closed by a plug or cap 23' and is adapted to be connected to the said end portion 16 of the Y shaped fitting 13 in the manner shown in fig. 2.

The third way or branch end 28 is normally closed by a plug or cap 29 (fig. 1) and is adapted to be connected to the outlet pipe 30, closed by a plug or cap 31, of a supply 32, in form of flexible bag, containing a liquid disinfectant.

The operative condition is shown in fig. 2, apart from the fracture membrane 27 the breaking of which takes place later on, as it will be appreciated from the following description of the operation of the device of the invention.

Starting from the condition of fig. 1 and on the assumption that a dialysis cycle is completed and the dialysis solution contained in the peritoneal cavity is to be discharged and substituted for by fresh dialysis solution, the caps 17 and 23' are removed and the two ed portions 16 and 23 are connected to each other in a seal tight manner.

In turn the cap 19 is removed and the branch 18 of the Y fitting 13 is connected to the disposal bag 21.

Then the caps 29 and 31 are removed and the branch 28 is connected to the bag 32 and thus to the supply of liquid disinfectant.

Consequently both the auxiliary fitting and the Y shaped fitting are washed by the liquid disinfectant which is partially directly discharged and then does fill the same fittings owing to the closing of the pipe 20 by means of a clamp 33 (of the same type of the clamp 12) and to the later closing of the pipe 30 by means of a like clamp 34.

Upon sufficient time is lapsed in order to have the disinfectant fulfilling its action, the clamp 33 on the pipe 20 is opened and the fracture membrane is broken, whereby the whole system is thoroughly washed by a predetermined amount of fresh dialysis solution (for example about 100 ml) and, by opening the said clamp 33 on the pipe 20, is directly discharged to the disposal bag 21.

Then a like clamp 35 provided on the pipe 25 is closed and the clamp 12 is opened whereby, the exhausted solution present in the peritoneal cavity (about 2 litres) is discharged to the disposal bag 21, thus improving the washing of the portion of the system closer to the catheter 11 from traces of the disinfectan.

Then the clamp on the pipe 20 is closed and the said clamp on the pipe 25 is opened whereby the fresh dialysis solution flows by gravity to the patient abdomen until the required amount fills it.

Lastly, the clamp 12 is closed, the clamp on the pipe 30 is opened and the disinfectant fills the fitting 13 the latter being thereafter disconnected from the fitting 22 and from the bag 21, the branches 16 and 18 being seal tight closed by means of plugs or caps 17 and 19 respectively. Thus the fitting 13 remains protected until the next cycle is carried out.

It is thus evident that the main object of the present invention is fully achieved in a simple and reliable manner.

The preceeding description is related to a preferred embodiment having no limiting purpose; changes shall be possible without falling out of the scope of the invention and will appear to the skilled man.

In this connection it is to be pointed out that the connnecting and disconnecting operations are carrried out in the well known manner for medical and therapeutical applications involving pipes and the like. Likewise the several component parts, as pipes, bags, clamps and so on, shall be manufactured from the well known plastic materials suitable for biomedical use, and shall be of flexible type.

A disinfecting auxiliary device for continuous ambulatorial peritoneal dialysis comprises a three way fitting, which can be fixed by a first branch to the end of the outlet pipe of a supply bag for fresh dialysis solution, a second branch being connectable to the end portion of the standard Y shaped fitting mounted to the catheter implanted in the patient abdomen and the third or side branch is connectable to a supply bag for liquid disinfectant.

## Claims

1. Disinfecting auxiliary device for peritoneal dialysis adapted to be mounted to the outlet of a supply bag containing fresh dialysis solution and connectable to the inlet of the standard Y shaped fitting fixed to the catheter implanted in the patient abdomen, characterized by comprising a three way fitting, one branch of which can be fixed to the outlet pipe of the said supply bag, a check valve being inserted in said one branch, an opposite second branch connectable to the inlet of said Y shaped fitting and a third, side branch connectable to a supply of liquid disinfectant, said second and third branches being provided with closing plugs.

2. Disinfecting auxiliary device according to claim 1, characterized in that said supply of disinfectant is a flexible bag provided with an outlet pipe, normally closed by a plug or cap.

3. Disinfecting auxiliary device according to claim 1, characterized in that said supply bag of fresh dialysis solution is a flexible bag provided with an outlet pipe normally closed by an end plug or cap.

4. Disinfecting auxiliary device according to claim 1, characterized in that said check valve is a fracture membrane.

*Fig. 1*

Fig. 2